(19) Europäisches Patentamt
European Patent Office
Office européen des brevets

(11) Veröffentlichungsnummer: **0 027 197**
**B1**

# (12) EUROPÄISCHE PATENTSCHRIFT

(45) Veröffentlichungstag der Patentschrift: **25.08.82**

(51) Int. Cl.³: **C 07 C 127/26**

(21) Anmeldenummer: **80105873.6**

(22) Anmeldetag: **27.09.80**

(54) **Verfahren zur Herstellung von Additionsverbindungen aus Carbodiimiden und Hydroxylgruppen aufweisenden Verbindungen.**

(30) Priorität: **11.10.79 DE 2941253**

(43) Veröffentlichungstag der Anmeldung: **22.04.81 Patentblatt 81/16**

(45) Bekanntmachung des Hinweises auf die Patenterteilung: **25.08.82 Patentblatt 82/34**

(84) Benannte Vertragsstaaten: **BE DE FR GB IT**

(56) Entgegenhaltungen:
**DE-C-956 499**
**US-A-3 347 390**
**JOURNAL OF THE CHEMICAL SOCIETY (C), Band 1967, Nr. 14 London A.J. BLOODWORTH et al. »Organometallic Reactions. Part VII. Further Addition Reactions of Tributyltin Methoxide and of Bistributyltin Oxide« Seiten 1309 bis 1313**

(73) Patentinhaber: **BAYER AG, Zentralbereich Patente, Marken und Lizenzen, D-5090 Leverkusen 1, Bayerwerk (DE)**

(72) Erfinder: **Schäfer, Walter, Dr., Am Wolfskaul 6, D-5000 Köln 80 (DE)**
Erfinder: **Wagner, Kuno, Dr., Am Kiesberg 8, D-5090 Leverkusen 1 (DE)**
Erfinder: **Findeisen, Kurt, Dr., In der Follmühle 10, D-5068 Odenthal (DE)**

**EP 0 027 197 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur Herstellung von Additionsverbindungen aus Carbodiimiden und Hydroxylgruppen aufweisenden Verbindungen

Die Erfindung betrifft ein verbessertes Verfahren zur Umsetzung von OH-Gruppen aufweisenden Verbindungen mit Carbodiimiden. Die Verbesserung besteht darin, daß Sn-II-Verbindungen als Katalysatoren für die Additionsreaktion eingesetzt werden.

Bekanntlich sind Additionsverbindungen aus äquivalenten Mengen an Hydroxylverbindungen und Carbodiimiden, insbesondere die Isoharnstoffether, wertvolle Textilhilfsstoffe und nützliche Stabilisatoren. Die Umsetzung von Alkoholen mit Carbodiimiden verläuft jedoch nur unter energischen Bedingungen, in der Regel bei Temperaturen von über 150° C. Aus DE-PS 956 499 ist zwar bekannt, daß die Umsetzung von Carbodiimiden mit Alkoholen zu Isoharnstoffethern durch Kupfersalze katalysiert wird; ein schwerwiegender Nachteil dieses Verfahrens ist jedoch, daß durch die Kupfersalze tiefgefärbte Rohprodukte entstehen, die für viele Anwendungszwecke erst einer Reinigungsprozedur unterworfen werden müssen, besonders dann, wenn sie als Textilhilfsstoffe eingesetzt werden sollen.

Aus dem Journal of the Chemical Society (C), 1967, No. 14, Seiten 1309–1313, ist ein Verfahren zur Umsetzung von Carbodiimiden mit Alkoholen bekannt, in dem als Katalysator zum Beispiel Tributylzinn-methoxid eingesetzt wird. Hinweise auf die Verwendung von Verbindungen des zweiwertigen Zinns finden sich in dieser Veröffentlichung nicht.

Aufgabe der vorliegenden Erfindung war es, ein verbessertes Verfahren aufzufinden, bei dem sich das Additionsprodukt aus Carbodiimid und Hydroxylverbindung nach der Umsetzung ohne Reinigungsprozedur für die angegebenen Zwecke einsetzen läßt.

Es wurde nun gefunden, daß man derartige Umsetzungsprodukte, insbesondere Isoharnstoffether und gegebenenfalls deren Umlagerungsprodukte, schon bei Temperaturen zwischen 25 und 140°C herstellen kann, wenn man Carbodiimid und Hydroxylverbindung in Gegenwart von katalytischen Mengen an Sn-II-Verbindungen umsetzt. Die Rohprodukte, die nach diesem erfindungsgemäßen Verfahren hergestellt werden, sind nicht verfärbt und können ohne Reinigungsprozedur eingesetzt werden. Dies ist von besonderem Vorteil, wenn das entstehende Produkt eine polymere Substanz ist.

Gegenstand der vorliegenden Erfindung ist somit ein Verfahren zur Herstellung von Additionsverbindungen aus Hydroxylgruppen aufweisenden Verbindungen und im wesentlichen von NCO-Gruppen freien Carbodiimiden, wobei die Reaktionspartner in Gegenwart von 0,01 bis 3 Gew.-%, bezogen auf Reaktionsgemisch, von Metallverbindungen als Katalysator, bevorzugt bei einer Temperatur zwischen 25 und 150° C, umgesetzt werden, welches dadurch gekennzeichnet ist, daß als Katalysator Verbindungen zweiwertigen Zinns eingesetzt werden.

Für das erfindungsgemäße Verfahren geeignete Sn-II-Verbindungen sind z. B. Salze mit $C_1-C_{25}$-Carbonsäuren wie Zinn-II-acetat, Zinn-II-oleat, Zinn-II-stearat, Zinn-II-octoat, Zinn-II-(2-ethylhexoat) und Zinn-II-laurat; $Sn(OH_2)$, $SnCl_2$, $SnF_2$, $SnBr_2$, $SnSO_4$ oder auch die durch die Auflösung von metallischem Zinn in anorganischen Säuren entstehenden Produkte.

Bevorzugt sind auch die Komplexverbindungen von Sn-II-Salzen, besonders solchen von organischen Säuren, mit Formamidinen und Guanidinen, z. B. der Komplex aus Sn-II-ethylhexoat und Tetramethylguanidin.

Als Ausgangsverbindungen zur Herstellung der Isoharnstoffether kommen z. B. Carbodiimide der Formel

$$R-N=C=N-R'$$

in welcher R und R' gleich oder verschieden sind und für einen (gegebenenfalls verzweigten) $C_1-C_{20}$-Alkylrest, $C_4-C_{15}$-Cycloalkylrest, $C_6-C_{15}$-Arylrest oder $C_7-C_{15}$-Aralkylrest stehen, in Frage, z. B. Diisopropylcarbodiimid, N-n-Butyl-N'-cyclohexylcarbodiimid, N,N'-Dicyclohexylcarbodiimid, N-tert.-Butyl-N'-cyclohexylcarbodiimid, N-Cyclohexyl-N'-phenylcarbodiimid, N-Methyl-N'-tert.-butylcarbodiimid, N-tert.-Butyl-N'-phenylcarbodiimid und N,N'-Diphenylcarbodiimid.

Weiterhin kommen in Frage die mit Hilfe von Phospholinoxid als Katalysator aus Polyisocyanaten zugänglichen Polycarbodiimide. Die Herstellung derartiger Polycarbodiimide wird z. B. in US 2 941 966 beschrieben. Die hierfür in Frage kommenden Polyisocyanate sind aliphatische, cycloaliphatische, araliphatische, aromatische und heterocyclische Polyisocyanate, wie sie z. B. von W. Siefken in Justus Liebigs Annalen der Chemie, 562, Seiten 75 bis 136, beschrieben werden, beispielsweise solcher der Formel

$$Q(NCO)_n$$

in der

n = 2–4, vorzugsweise 2, und
Q einen aliphatischen Kohlenwasserstoffrest mit 2–18, vorzugsweise 6–10 C-Atomen,
einen cycloaliphatischen Kohlenwasserstoffrest mit 4–15, vorzugsweise 5–10 C-Atomen,
einen aromatischen Kohlenwasserstoffrest mit 6–15, vorzugsweise 6–13 C-Atomen oder
einen araliphatischen Kohlenwasserstoffrest mit 8–15, vorzugsweise 8–13 C-Atomen,

bedeuten, z. B. Äthylen-diisocyanat, 1,4-Tetramethylendiisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethyl-cyclohexan (DE-Auslegeschrift 1 202 785, US-Patentschrift 3 401 190), 2,4- und 2,6-Hexahydrotoluylendiisocyanat sowie beliebige Gemische dieser Isomeren, Hexahydro-1,3- und/oder -1,4-phenylendiisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Es ist prinzipiell auch möglich, aliphatische oder aromatische Diisocyanate zu verwenden oder mitzuverwenden, wie sie durch Umsetzung von überschüssigem Diisocyanat mit difunktionellen OH- oder NH-Gruppen tragenden Verbindungen entstehen und in der Praxis der Polyurethanchemie entweder als »modifizierte Isocyanate« oder als »Isocyanatpräpolymere« anzutreffen sind.

Bei der Herstellung des Polycarbodiimids wird zu dessen Molekulargewichtsregulierung dem Reaktionsgemisch aus Polyisocyanat und Phospholinoxid vorzugsweise noch ein Kettenabbrecher in einem Molverhältnis (bezüglich Polyisocyanat) kleiner als 1 : 1 zugegeben, z. B. Methanol, Ethanol, Cyclohexanol, Ethylamin, Anilin, Phenyl- oder Tolylisocyanat.

Als zweite Komponente zur Herstellung der Isoharnstoffether kommen Hydroxylverbindungen der allgemeinen Formel

$$R^1\text{-}(OH)_m$$

in Betracht, wobei der Rest $R^1$ aliphatisch, cycloaliphatisch, araliphatisch oder aromatisch, aber auch von heterocyclischer Natur sein kann, und m eine ganze Zahl, vorzugsweise von 1 bis 10, besonders bevorzugt von 1 bis 4, darstellt.

Aliphatische Reste $R^1$ sind geradkettige oder verzweigte, gesättigte oder ungesättigte Kohlenwasserstoffreste mit vorzugsweise 1 bis 25 Kohlenstoffatomen, welche bis zu 2 Doppel- oder eine Dreifachbindung enthalten können, cycloaliphatische Reste, solche mit 5, 6, 7, 8, 10 oder 12 Kohlenstoffatomen im Ringsystem, die gegebenenfalls auch substituiert sein können, beispielsweise durch $C_6-C_{20}$-Arylreste, bevorzugt einen Phenyl- und Naphthylrest, durch $C_6-C_{10}$-Aroxyreste, bevorzugt einen Phenoxyrest, durch $C_1-C_6$-Alkoxyreste, Nitrogruppen, Halogen-, (Fluor, Chlor, Brom, Jod), $C_1-C_6$-Acyl-, $C_1-C_6$-Alkylmercapto-, $C_6-C_{20}$-Arylmercapto-, Cyano-, $C_1-C_{12}$-Carbonester-, Carboamid-, $C_1-C_8$-Dialkyl-amino- oder $C_1-C_6$-Acylamino-Reste.

Als aromatische Reste $R^1$ kommen aromatische Kohlenwasserstoffreste mit bis zu 20 Kohlenstoffatomen, vorzugsweise mit 6 bis 14 Kohlenstoffatomen, insbesondere mit bis zu 10 Kohlenstoffatomen (besonders bevorzugt Phenyl- und Naphthylreste) im Ringsystem in Betracht. Die aromatischen Reste $R^1$ können z. B. folgende Substituenten aufweisen: Alkyl-, Dialkylamino- und Alkoxyreste mit 1 bis 12 Kohlenstoffatomen, vorzugsweise mit 1 bis 6 Kohlenstoffatomen, Aryl- und Aroxygruppen, vorzugsweise Phenyl- und Phenoxyreste, Halogen (vorzugsweise Fluor, Chlor, Brom), $-CHO$, Carbonestergruppen, z. B. mit aliphatischen Resten mit 1 bis 12, vorzugsweise 1 bis 6 C-Atomen, Phenyl- oder Benzylresten, Carbonamidgruppen und Alkylsulfonylgruppen mit 1 bis 12, vorzugsweise 1 bis 6 C-Atomen, Arylsulfonylgruppen (vorzugsweise Phenylsulfonyl), Sulfonestergruppen, z. B. mit aliphatischen Resten mit 1 bis 12, vorzugsweise 1 bis 6 C-Atomen, Phenyl oder Benzyl, Sulfonamidgruppen, Acylgruppen, z. B. mit aliphatischen Resten mit 2 bis 12, vorzugsweise 2 bis 6 C-Atomen oder Benzoylgruppen, Cyano-, Alkylmercapto- oder Acylmercaptogruppen mit 1 bis 12, vorzugsweise 2 bis 6 C-Atomen, Arylmercapto-(vorzugsweise Phenylmercapto-)Gruppen sowie 1 bis 2 $NO_2$-Gruppen.

Als heterocyclische Reste $R^1$ kommen vorzugsweise 5-, 6- oder 7gliedrige Ringsysteme mit einem oder mehreren Heteroatomen wie O, N oder S im Ringsystem in Betracht.

Ferner kommen als Verbindungen

$$R^1\text{-}(OH)_m$$

Phenole und Alkohole mit mehreren OH-Gruppen in Betracht, wie Hydrochinon, Brenzcatechin, Resorcin, Cellulose, Äthylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bis-hydroxymethyl-cyclohexan, 2-Methyl-1,3-propandiol, Dibrombutendiol (US-Patentschrift 3 723 392), Glyzerin, Trimethylolpropan, Hexantriol-(1,2,6), Trimethyloläthan, Pentaerythrit, Chinit, Mannit und Sorbit, Ricinusöl, Diäthylenglykol, Triäthylenglykol, Tetraäthylenglykol, höhere Polyäthylenglykole mit einem Molekulargewicht bis 400, Dipropylenglykol, höhere Polypropylenglykole mit einem Molekulargewicht bis 400, Dibutylenglykol, höhere Polybutylenglykole mit einem Molekulargewicht bis 400, 4,4'-Dihydroxydiphenylpropan, Di-hydroxymethyl-hydrochinon, Ethanolamin, Diethanolamin, N-Methyldiethanolamin, Triethanolamin und 3-Aminopropanol.

Als niedermolekulare Polyole kommen erfindungsgemäß auch die Gemische von Hydroxyaldehyden und Hydroxyketonen (»Formose«) bzw. die hieraus durch Reduktion erhaltenen mehrwertigen Alkohole (»Formit«) in Frage, wie sie bei der Selbstkondensation von Formaldehydhydrat in Gegenwart von Metallverbindungen als Katalysator und von zur Endiolbildung befähigten Verbindungen als Co-Katalysator entstehen (DE-Offenlegungsschriften 2 639 084, 2 714 084, 2 714 104, 2 721 186, 2 738 154 und 2 738 512).

Als Reaktionspartner für das Carbodiimid kommen erfindungsgemäß aber auch höhermolekulare Polyhydroxylverbindungen in Betracht, insbesondere zwei bis acht Hydroxylgruppen aufweisende Verbindungen, speziell solche vom Molekulargewicht 800 bis 10 000, vorzugsweise 1000 bis 6000, z. B. mindestens zwei, in der Regel 2 bis 8, vorzugsweise aber 2 bis 4 Hydroxylgruppen aufweisende Polyester, Polyether, Polythioether, Polyacetale, Polycarbonate und Polyesteramide, wie sie für die Herstellung von homogenen und von zellförmigen Polyurethanen an sich bekannt sind:

Die in Frage kommenden, Hydroxylgruppen aufweisenden Polyester sind z. B. Umsetzungsprodukte von mehrwertigen, vorzugsweise zweiwertigen und gegebenenfalls zusätzlich dreiwertigen Alkoholen mit mehrwertigen, vorzugsweise zweiwertigen Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z. B. durch Halogenatome, substituiert und/oder ungesättigt sein.

Als Beispiele für solche Carbonsäuren und deren Derivate seien genannt:

Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimerisierte und trimerisierte ungesättigte Fettsäuren, gegebenenfalls in Mischung mit monomeren ungesättigten Fettsäuren, wie Ölsäure; Terephthalsäurediemethylester und Terephthalsäure-bis-glykolester. Als mehrwertige Alkohole kommen z. B. Äthylenglykol, Propylenglykol-(1,2) und -(1,3), Butylenglykol-(1,4) und -(2,3), Hexandiol-(1,6), Octandiol-(1,8), Neopentylglykol, 1,4-Bis-hydroxymethylcyclohexan, 2-Methyl-1,3-propandiol, Glycerin, Trimethylolpropan, Hexantriol-(1,2,6), Butantriol-(1,2,4), Trimethyloläthan, Pentaerythrit, Chinit, Mannit und Sorbit, Formit, Methylglykosid, ferner Diäthylenglykol, Triäthylenglykol, Tetraäthylenglykol und höhere Polyäthylenglykole, Dipropylenglykol und höhere Polypropylenglykole sowie Dibutylenglykol und höhere Polybutylenglykole in Frage. Die Polyester können anteilig endständige Carboxylgruppen aufweisen. Auch Polyester aus Lactonen, z. B. ε-Caparolacton, oder aus Hydroxycarbonsäuren, z. B. ω-Hydroxycapronsäure, sind einsetzbar.

Auch die erfindungsgemäß in Frage kommenden, mindestens zwei, in der Regel zwei bis acht, vorzugsweise zwei bis drei Hydroxylgruppen aufweisenden Polyäther sind solche der an sich bekannten Art und werden z. B. durch Polymerisation von Epoxiden wie Äthylenoxid, Propylenoxid, Butylenoxid, Tetrahydrofuran, Styroloxid oder Epichlorhydrin mit sich selbst, z. B. in Gegenwart von Lewis-Katalysatoren wie $BF_3$, oder durch Anlagerung dieser Epoxide, vorzugsweise von Äthylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z. B. Äthylenglykol, Propylenglykol-(1,3) oder -(1,2), Trimethylolpropan, Glycerin, Sorbit, 4,4'-Dihydroxy-diphenylpropan, Anilin, Äthanolamin oder Äthylendiamin hergestellt. Auch Sucrosepolyäther, wie sie z. B. in den DE-Auslegeschriften 1 176 358 und 1 064 938 beschrieben werden, sowie auf Formit oder Formose gestartete Polyäther (DE-Offenlegungsschriften 2 639 083 bzw. 2 737 951), kommen erfindungsgemäß in Frage. Vielfach sind solche Polyäther bevorzugt, die überwiegend (bis zu 90 Gew.-%, bezogen auf alle vorhandenen OH-Gruppen im Polyäther) primäre OH-Gruppen aufweisen. Auch OH-Gruppen aufweisende Polybutadiene sind erfindungsgemäß geeignet.

Unter den Polythioäthern seien insbesondere die Kondensationsprodukte von Thiodiglykol mit sich selbst und/oder mit anderen Glykolen, Dicarbonsäuren, Formaldehyd, Aminocarbonsäuren oder Aminoalkoholen angeführt. Je nach den Co-Komponenten handelt es sich bei den Produkten z. B. um Polythiomischäther, Polythioätherester oder Polythioätheresteramide.

Als Polyacetale kommen z. B. die aus Glykolen, wie Diäthylenglykol, Triäthylenglykol, 4,4'-Dioxäthoxydiphenyldimethylmethan, Hexandiol und Formaldehyd herstellbaren Verbindungen in Frage. Auch durch Polymerisation cyclischer Acetale wie z. B. Trioxan (DE-Offenlegungsschrift 1 694 128) lassen sich erfindungsgemäß geeignete Polyacetale herstellen.

Als Hydroxylgruppen aufweisende Polycarbonate kommen solche der an sich bekannten Art in Betracht, die z. B. durch Umsetzung von Diolen wie Propandiol-(1,3), Butandiol-(1,4) und/oder Hexandiol-(1,6), Diäthylenglykol, Triäthylenglykol, Tetraäthylenglykol oder Thiodiglykol mit Diarylcarbonaten, z. B. Diphenylcarbonat, oder Phosgen hergestellt werden können (DE-Auslegeschriften 1 694 080, 1 915 908 und 2 221 751; DE-Offenlegungsschrift 2 605 024).

Zu den Polyesteramiden und Polyamiden zählen z. B. die aus mehrwertigen gesättigten oder ungesättigten Carbonsäuren bzw. deren Anhydriden und mehrwertigen gesättigten oder

ungesättigten Aminoalkoholen, Diaminen, Polyaminen und deren Mischungen gewonnenen, vorwiegend linearen Kondensate.

Auch bereits Urethan- oder Harnstoffgruppen enthaltende Polyhydroxylverbindungen sowie gegebenenfalls modifizierte natürliche Polyole, wie Rizinusöl oder Kohlenhydrate, z. B. Stärke, sind verwendbar. Auch Anlagerungsprodukte von Alkylenoxiden an Phenol-Formaldehyd-Harze oder auch an Harnstoff-Formaldehyd-Harze sind erfindungsgemäß einsetzbar.

Vertreter der genannten erfindungsgemäß zu verwendenden Verbindungen sind z. B. in High Polymers, Vol. XVI, »Polyurethanes, Chemistry and Technology«, verfaßt von Saunders-Frisch, Interscience Publishers, New York, London, Band I, 1962, Seiten 32–42 und Seiten 44–54, und Band II, 1964, Seiten 5–6 und 198–199, sowie im Kunststoff-Handbuch, Band VII, Vieweg-Höchtlen, Carl-Hanser-Verlag, München, 1966, z. B. auf den Seiten 45–71, beschrieben.

Ferner kommen als Hydroxylkomponenten auch Polyhydroxyorganopolysiloxane in Frage, z. B.

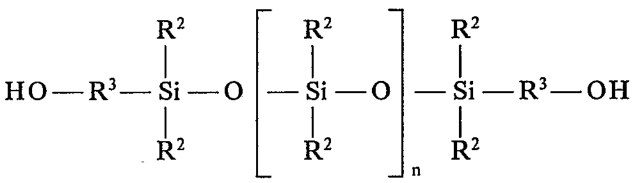

wobei $R^3$ für einen zweiwertigen Kohlenwasserstoffrest mit 1 bis 6 C-Atomen und $R^2$ für einen einwertigen Alkylrest mit bis 4 C-Atomen steht und n eine ganze Zahl von 0 bis 10 000 bedeutet.

Als Komponente $R^1-OH$ kommen auch solche Verbindungen in Betracht, in denen $R^1$ ein substituiertes Phosphorradikal ist. Bevorzugte derartige Radikale sind Dialkoxyphosphinradikale wie das Dimethoxyphosphin-, Diethoxyphosphin-, Dipropoxyphosphin-, Dibutoxyphosphin-, Bis-(2,2,2-trifluorethoxy)-phosphin-, Bis-(2-hydroxypropoxy)-phosphin-, Bis-(2-chlorethoxy)-phosphin-, Bis-(2-fluorethoxy)-phosphin- und Diphenoxyphosphinradikal.

Das erfindungsgemäße Verfahren wird vorzugsweise so durchgeführt, daß die Hydroxylverbindung und das Carbodiimid im gewünschten Molverhältnis (vorzugsweise in etwa äquivalenten Mengen) vereinigt werden und anschließend die Sn-Verbindung bevorzugt in Mengen von $^1/_{10}$ bis $^1/_{1000}$ Mol, bezogen auf 1 Mol Carbodiimid, zugesetzt wird. Wenn bei Raumtemperatur noch keine Reaktion festzustellen ist, was sich leicht in einem Vorversuch anhand des Verschwindens der Carbodiimidbande (2120 $cm^{-1}$) im IR-Spektrum überprüfen läßt, wird das Reaktionsgemisch langsam erhitzt, wobei jedoch 140° C vorzugsweise nicht überschritten werden. Die so erhaltenen Rohprodukte an Isoharnstoffether zeigen keine Verfärbung und benötigen keine Reinigung.

Die Reaktion kann sowohl in Substanz als auch in einem gegenüber Carbodiimid und Hydroxylverbindung inerten Lösungsmittel durchgeführt werden.

Geeignete Lösungsmittel sind z. B.: Toluol, o- und m-Dichlorbenzol, Nitrobenzol, Methylenchlorid, Essigester, Aceton, Methylethylketon, Acetonitril, Tetrahydrofuran, Dioxan, Dimethylformamid, Dimethylsulfoxid, Sulfolan und Hexamethylphosphorsäuretriamid.

Sowohl die Mono- als auch die Polyisoharnstoffether eignen sich z. B. als Stabilisatoren für Polyester (DE-OS 2 528 589) und Textilhilfsmittel (DE-PS 1 011 869). Die Verfahrenserzeugnisse können aber auch als Zwischenprodukte für weitere Synthesen, z. B. von Pflanzenschutzmitteln und Arzneimitteln, von Bedeutung sein. Darüber hinaus stellen Isoharnstoffether Alkylierungsmittel dar (THL [1974] 143) und sind Vorstufen zur Olefinisierung von Hydroxylverbindungen (Chimia 29, 520 [1975]).

## Beispiel 1

194 g Diphenylcarbodiimid werden mit 46 g Ethanol versetzt, und die Lösung wird auf 60° C aufgeheizt. Nachdem der Reaktionslösung 0,05 g Sn-II-(2-ethylhexoat) zugesetzt werden, entsteht innerhalb von 3 Stunden quantitativ der Isoharnstoff. Im IR-Spektrum verschwindet die Carbodiimidbande bei 2120 $cm^{-1}$, stattdessen erscheint die Bande des Isoharnstoffethers bei 1655 $cm^{-1}$.

$Sdp._{0,07} = 122°$ C.

## Beispiel 2
(Vergleich)

194 g Diphenylcarbodiimid werden mit 46 g Ethanol versetzt, und die Lösung wird anschließend auf 60° C aufgeheizt. Nach 3 Stunden ist noch keine Umsetzung festzustellen.

## Beispiel 3

32 g Methanol, 206 g Dicyclohexylcarbodiimid und 0,1 g Desmorapid Sn-II-(2-ethylhexoat) werden zusammengegeben, und das Reaktionsgemisch wird 2 Stunden lang auf 80°C aufgeheizt, wobei die Carbodiimidbande (2120 $cm^{-1}$) völlig verschwindet.

Aus dem Rohprodukt lassen sich bei 102 bis 104°C unter einem Druck von 0,1 Torr 214 g (89% der Theorie) des Isoharnstoffethers abdestillieren.

## Beispiel 4

194 g Diphenylcarbodiimid, 31 g Ethylenglykol und 0,05 g Sn-II-(2-ethylhexoat) werden in 200 g Xylol gelöst. Die Reaktionslösung wird auf Rückflußtemperatur gebracht, wobei Diphenylharnstoff ausfällt. Nach dem Einengen der Xylol-Lösung erhält man 102 g (86% der Theorie) 2-Phenylimino-3-phenyl-oxazolidin.
Smp.: 115 bis 117°C.

## Beispiel 5

222 g Ditolylcarbodiimid, 1000 g eines linearen Polypropylenglykols (OH-Zahl 56) und 0,1 g Sn-II-(2-ethylhexoat) werden zusammengerührt. Das Reaktionsgemisch wird auf 120°C aufgeheizt. Im IR-Spektrum verschwindet die Carbodiimidbande, und es entsteht das Bis-(N,N'-ditolylisoharnstoff)-derivat des Polypropylenoxiddiols.

## Beispiel 6

16,7 g Bis-(3,4-dichlorphenyl)-carbodiimid, 5,9 g Benzylalkohol, 60 ml Toluol und 0,1 g Sn-II-(2-ethylhexoat) werden zusammengegeben, und das Reaktionsgemisch wird 30 Minuten lang auf 70°C aufgeheizt. Nach dem Einengen der Reaktionsmischung kristallisiert der O-Benzyl-N,N'-di-(3,4-dichlorphenyl)-isoharnstoff nahezu quantitativ aus.
Smp.: 95 bis 97°C.

## Beispiel 7

10,3 g Dicyclohexylcarbodiimid, 5,35 g Cyclohexanol, 60 ml Xylol und 0,1 g Sn-II-(2-ethylhexoat) werden zusammengegeben, und das Reaktionsgemisch wird 2 Stunden lang auf 130°C aufgeheizt, wobei die Carbodiimidbande bei 2120 $cm^{-1}$ völlig verschwindet. Nach dem Einengen der Reaktionsmischung kristallisiert der O,N,N'-Tricyclohexylisoharnstoff aus.
Smp.: 55 bis 57°C.

## Beispiel 8

a) 12,7 g Di-p-tolylcarbodiimid, 5,18 g Phenol, 60 ml Toluol und 0,1 g Sn-II-(2-ethylhexoat) werden zusammengegeben, und das Reaktionsgemisch wird 2 Stunden auf 110°C aufgeheizt, wobei die Carbodiimidbande bei 2120 $cm^{-1}$ völlig verschwindet. Nach dem Einengen der Reaktionsmischung kristallisiert der O-Phenyl-N,N'-di-p-tolylisoharnstoff aus.
Smp.: 112 bis 113°C.

b) Wird vergleichsweise das in a) dargestellte Gemisch ohne Sn-II-(2-ethylhexoat) erwärmt, so tritt keine merkliche Reaktion ein und die Carbodiimidbande bei 2120 $cm^{-1}$ bleibt bestehen.

## Beispiel 9

a) 19,4 g Diphenylcarbodiimid, 18 g Diethylphosphit und 0,1 g Sn-II-(2-ethylhexoat) werden zusammengegeben, und die Reaktionsmischung wird 1,5 Stunden auf 100°C aufgeheizt. Nach dem Erkalten kristallisiert das 1 : 1-Addukt aus, das aus Petrolether umkristallisiert eine Smp. von 112°C hat.

b) Wird das in a) dargestellte Gemisch ohne Sn-II-(2-ethylhexoat) auf 100°C erwärmt, so ist keine Umsetzung festzustellen.

Beispiel 10

In eine Mischung von 8,9 g (0,1 Mol) Dimethylethanolamin und 0,05 g Sn-II-(2-ethylhexoat) in 100 ml getrocknetem Toluol wird eine Lösung von 33,2 g (0,1 Mol) Bis-[3,4-dichlorphenyl]-carbodiimid in 100 ml Toluol unter Rühren bei 30°C zugetropft. Die Reaktion verläuft exotherm. Das Reaktionsgemisch wird weitere 30 Minuten bei 60°C gerührt. Nachdem flüchtige Verbindungen im Hochvakuum (0,05 Torr) bei 60°C abgezogen wurden, erhält man in nahezu quantitativer Ausbeute den Isoharnstoff in Form weißer Kristalle vom Schmelzpunkt 76°C.

Beispiel 11

14,6 g (0,1 Mol) Dianhydrosorbit, 66,4 g (0,2 Mol) Bis-[3,4-dichlorphenyl]-carbodiimid und 0,05 g Sn-II-(2-ethylhexoat) werden in 200 ml Dioxan gelöst. Das Reaktionsgemisch wird 5 Stunden lang bei 80°C gerührt und anschließend bei einem Vakuum von 15 Torr vom Lösungsmittel befreit. Nach dem Waschen des Rohproduktes mit Toluol werden 52 g Kristalle des Isoharnstoffes vom Schmelzpunkt 132°C isoliert.

Beispiel 12

56 g (0,1 Mol) Polyäthylenglykol, 38,8 g (0,2 Mol) Diphenylcarbodiimid und 0,05 g Sn-II-(2-ethylhexoat) werden auf 100°C aufgeheizt. Das entstehende Isoharnstoffderivat ist ein farbloses Harz mit einer Viskosität von 50 Pa · s bei 30°C.

Beispiel 13

50 g eines Polycarbodiimids auf Basis von Isophorondiisocyanat mit durchschnittlich ca. 4 Carbodiimidgruppen pro Molekül werden zusammen mit 0,05 g Sn-II-(2-ethylhexoat) in 100 ml Äthanol gelöst und zum Rückfluß erhitzt. Nachdem die Carbodiimidabsorption bei 2120 $cm^{-1}$ verschwunden ist, wird die Polyisoharnstofflösung vom Lösungsmittel befreit. Das trockene Produkt hat einen Schmelzbereich von 61 – 65°C.

Das Ausgangsmaterial (Polycarbodiimid) wird durch 2½stündiges Erwärmen von Isophorondiisocyanat unter Katalyse von 1 Gewichtsprozent 1-Methyl-1-phospha-cyclopentan-1-oxid (»Phospholinoxid«) auf 150 – 160°C erhalten.

**Patentansprüche**

1. Verfahren zur Herstellung von Additionsverbindungen aus Hydroxylgruppen aufweisenden Verbindungen und im wesentlichen von NCO-Gruppen freien Carbodiimiden, wobei die Reaktionspartner in Gegenwart von 0,01 bis 3 Gew.-%, bezogen auf Reaktionsgemisch, von Metallverbindungen als Katalysator umgesetzt werden, dadurch gekennzeichnet, daß als Katalysator Verbindungen des zweiwertigen Zinns eingesetzt werden.
2. Verfahren nach Anspruch 1, dadurch gekennzeichnet, daß Salze des zweiwertigen Zinns mit $C_1 - C_{25}$-Carbonsäuren eingesetzt werden.
3. Verfahren nach Anspruch 2, dadurch gekennzeichnet, daß als Katalysator Sn-II-(2-ethylhexoat) eingesetzt wird.
4. Verfahren nach Anspruch 1 bis 3, dadurch gekennzeichnet, daß die Umsetzung im Temperaturbereich zwischen 25 und 150°C vorgenommen wird.

**Claims**

1. Process for the production of addition compounds of compounds containing hydroxyl groups and carbodiimides substantially free from NCO groups, the reactants being reacted in the presence of 0.01 to 3% by weight, based on the reaction mixture, of metal compounds as the catalyst, characterised in that compounds of divalent tin are used as the catalyst.
2. Process according to Claim 1, characterised in that salts of divalent tin with $C_1 - C_{25}$ carboxylic acids are used.
3. Process according to Claim 2, characterised in that Sn(II)-(2-ethyl hexoate) is used as the catalyst.
4. Process according to Claim 1 to 3, characterised in that the reaction is carried out in the temperature range between 25 and 150°C.

**Revendications**

1. Procédé de préparation de composés d'addition de composés portant des groupes hydroxy et de carbodiimides essentiellement exempts de groupes NCO, dans lequel on fait réagir les composants en présence de 0,01 à 3% en poids, par rapport au mélange de réaction, de composés métalliques qui servent de catalyseurs, caractérisé en ce que l'on utilise en tant que catalyseurs des composés de l'étain divalent.

2. Procédé selon la revendication 1, caractérisé en ce que l'on utilise des sels de l'étain divalent et d'acides carboxyliques en $C_1 - C_{25}$.

3. Procédé selon la revendication 2, caractérisé en ce que l'on utilise en tant que catalyseur le 2-éthylhexanoate de Sn-II.

4. Procédé selon les revendications 1 à 3, caractérisé en ce que la réaction est effectuée dans un intervalle de températures de 25 à 150° C.